# EUROPÄISCHE PATENTSCHRIFT

(11) **EP 1 992 297 B1**
(45) Veröffentlichungstag und Bekanntmachung des Hinweises auf die Patenterteilung: **15.07.2009**
(21) Anmeldenummer: 08156098.9
(22) Anmeldetag: 13.05.2008
(51) Int. Cl.: A61B 17/17

(54) **Markraumnagel mit Zielkörper**
Medullary nail with guide
Clou d'ostéosynthèse intramédullaire doté d'un corps de guidage

(30) Priorität: 15.05.2007 DE 102007022648
(43) Veröffentlichungstag der Anmeldung: 19.11.2008
(73) Patentinhaber: Aesculap AG, 78532 Tuttlingen (DE)
(72) Erfinder: Wimber, Johannes, 78532 Tuttlingen (DE)
(74) Vertreter: Hoeger, Stellrecht & Partner Patentanwälte

(56) Entgegenhaltungen:
- WO-A-92/01422
- DE-A1- 19 806 323
- US-A- 5 374 271

## Beschreibung

Die Erfindung betrifft einen Markraumnagel mit einem in den Markraum eines Röhrenknochens einsetzbaren Schaft, der mehrere diametrale Durchgangsbohrungen mit unterschiedlicher Winkellage in Bezug auf die Längsachse des Schaftes aufweist, und mit einem über eine Halterung mit dem Schaft verbundenen, im Abstand zu diesem angeordneten Zielkörper, der Führungskanäle für ein Werkzeug aufweist, die mit jeweils einer Durchgangsbohrung des Schaftes ausgerichtet sind.

Markraumnägel weisen in der Regel mehrere, in unterschiedlicher Winkellage relativ zur Längsachse eines Schaftes verlaufende Durchgangsbohrungen auf, durch die Verriegelungsschrauben hindurch gesteckt werden können, die in den Knochen eingeschraubt werden. Um die Verriegelungsschrauben einschrauben zu können, müssen entsprechende Bohrungen in den Knochen eingebracht werden, und diese Bohrungen müssen mit den Durchgangsbohrungen des Schaftes ausgerichtet sein, der in dem Markraum des Röhrenknochens eingesetzt ist. Zu diesem Zweck ist es bekannt, Markraumnägel mit Zielkörpern zu verbinden, die außerhalb des Röhrenknochens angeordnet sind und die Führungskanäle aufweisen, durch die ein Werkzeug, beispielsweise ein Knochenbohrer, hindurch geschoben werden kann. Diese Führungskanäle müssen mit entsprechenden Durchgangsbohrungen des Schaftes ausgerichtet sein, und dazu sind zum Teil sehr platzaufwändige Konstruktionen notwendig, da die Durchgangsbohrungen im Schaft eine unterschiedliche Winkellage einnehmen, also in unterschiedlichen Richtungen laufen. So ist es beispielsweise bekannt, halbkreisförmige Zielkörper zu verwenden, die im Wesentlichen koaxial zur Längsachse des Schaftes verlaufen und längs ihres Umfanges Führungskanäle aufweisen, die mit den entsprechenden Durchgangsbohrungen des Schaftes ausgerichtet sind.

Dokument US-A-5,374,271 offenbart einen Markraumnagel mit einem Zielkörper nach dem Oberbegriff des Anspruchs 1.

Es ist Aufgabe der Erfindung, einen Markraumnagel der gattungsgemäßen Art so auszubilden, dass er Platz sparend ausgebildet ist und trotzdem dem Benutzer die Möglichkeit bietet, ein Werkzeug geführt mit unterschiedlichen Durchgangsbohrungen des Schaftes auszurichten.

Diese Aufgabe wird bei einem Markraumnagel der eingangs beschriebenen Art erfindungsgemäß dadurch gelöst, dass die Halterung des Schaftes um die Längsachse des Schaftes drehbar mit diesem und um eine parallele Drehachse drehbar mit dem Zielkörper verbunden ist, dass Getriebemittel vorgesehen sind, die bei einer Drehung der Halterung relativ zum Schaft den Zielkörper synchron relativ zur Halterung um denselben Drehwinkel verdrehen, so dass der Schaft und der Zielkörper bei allen Winkelstellungen der Halterung immer zueinander eine unveränderte Winkelstellung beibehalten, und dass die Führungskanäle in dem Zielkörper winkelmäßig gleich orientiert sind wie die Durchgangsbohrungen in dem Schaft.

Diese Ausgestaltung macht es möglich, durch die Drehung der Halterung den Zielkörper immer in dieselbe Ebene zu drehen, in der die Durchgangsbohrung verläuft, zu der ein Führungskanal ausgerichtet werden soll. Sobald der Zielkörper in dieser Ebene steht, ist der entsprechende Führungskanal automatisch mit der Durchgangsbohrung des Schaftes ausgerichtet, da der Schaft und der Zielkörper immer die gleiche Winkellage einnehmen, und dies gilt natürlich auch für die Durchgangsbohrungen und die Führungskanäle. Der Benutzer weiß außerdem sofort, welchen der Führungskanäle er benutzen muss, um eine mit einem Führungskanal ausgerichtete Durchgangsbohrung zu treffen, denn von den unterschiedlich orientierten Führungskanälen ist jeweils nur ein Führungskanal genau in Richtung auf den Schaft ausgerichtet, und dies ist der einzige Führungskanal, der bei einer bestimmten Stellung der Halterung mit einer Durchgangsbohrung ausgerichtet ist. Natürlich ist es möglich, dass im Schaft mehrere Durchgangsbohrungen angeordnet sind, die in derselben Ebene liegen, die sich aber in ihrer Höhe oder in ihrer Neigung gegenüber der Längsachse des Schaftes unterscheiden, dann sind auch im Zielkörper entsprechende Führungskanäle in einer Ebene angeordnet. In diesem Fall muss der Benutzer selbstverständlich unter den in der gleichen Ebene liegenden und allesamt auf den Schaft aufweisenden Führungskanälen den auswählen, der mit der von ihm gewünschten Durchgangsbohrung fluchtet.

Die Getriebemittel, die bei einer Verdrehung der Halterung gegenüber dem Schaft den Zielkörper synchron verdrehen, können sehr verschieden ausgebildet sein. Beispielsweise kann es sich dabei um einen Riemen handeln, der an der Halterung angeordnet ist und um Riemenumlenkflächen herumläuft, die drehfest mit dem Schaft bzw. dem Zielkörper verbunden sind.

Der Begriff "Riemen" kann dabei die unterschiedlichsten flexiblen Antriebsmittel umfassen, beispielsweise Zahnriemen, deren Zähne mit Zähnen auf der Riemenumlenkfläche kämmen, Seilzüge, die um Seilscheiben umlaufen, Ketten, die mit den Zähnen von Kettenrädern kämmen, einen Kugeldraht, dessen Kugeln in entsprechende Ausnehmungen auf den Riemenumlenkflächen eingreifen. Der Begriff "Riemen" und "Riemenumlenkflächen" ist daher in diesem Sinne umfassend zu verstehen als flexibler Umschlingungstrieb mit entsprechenden Umlenkflächen.

Bei einer anderen Ausführungsform kann vorgesehen sein, dass die Getriebemittel miteinander kämmende Zahnkränze umfassen, beispielsweise können in der Halterung mehrere um parallele Drehachsen verdrehbare Zahnräder hintereinander angeordnet sein, die die synchrone Verdrehung hervorrufen.

Vorteilhaft ist es, wenn die Halterung ein Gehäuse ausbildet, in dem die Getriebemittel angeordnet sind. Dadurch sind die Getriebemittel vollständig nach außen hin geschützt, so dass jede Verletzungsgefahr vermieden wird.

Günstig ist es, wenn der Zielkörper die Form eines Stabes hat, dessen Längsachse mit der Drehachse des Zielkörpers relativ zu der Halterung zusammenfällt. Dadurch ergibt sich eine besonders Platz sparende Anordnung, ein solcher Stab hat eine sehr ähnliche Form und ein sehr ähnliches Aussehen wie der Schaft, und dies erleichtert dem Benutzer auch, die gewünschten Führungskanäle auszuwählen, die sehr ähnlich angeordnet sind wie die Durchgangsbohrungen in dem Schaft.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Ansicht eines Markraumnagels mit einem Schaft und einem stabförmigen Zielkörper sowie einem Zahnriementrieb zum synchronen Verdrehen von Zielkörper und Schaft;
- Figur 2:: eine vergrößerte Ansicht des Markraumnagels der Figur 1 in einer anderen Relativstellung der Halterung und des Zielkörpers zum Schaft und

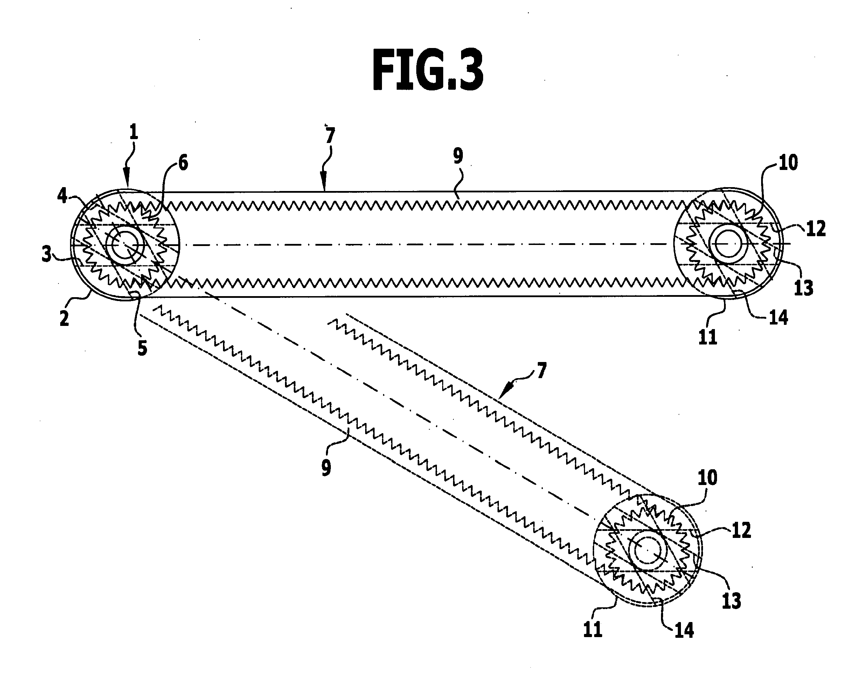

- Figur 3:: eine Draufsicht auf die Halterung des Markraumnagels der Figuren 1 und 2 mit unterschiedlicher Stellung der Halterung relativ zum Schaft.

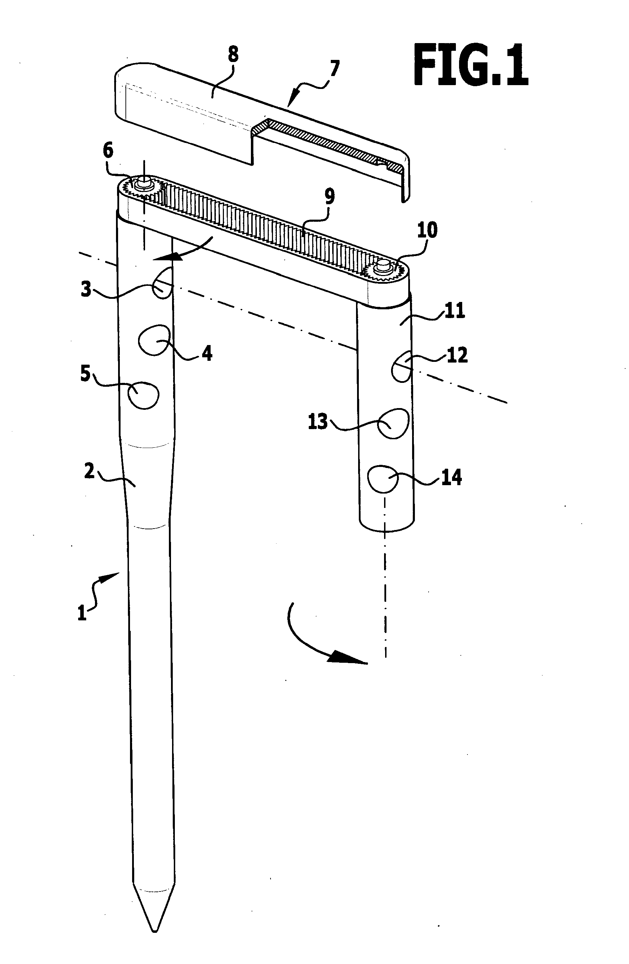

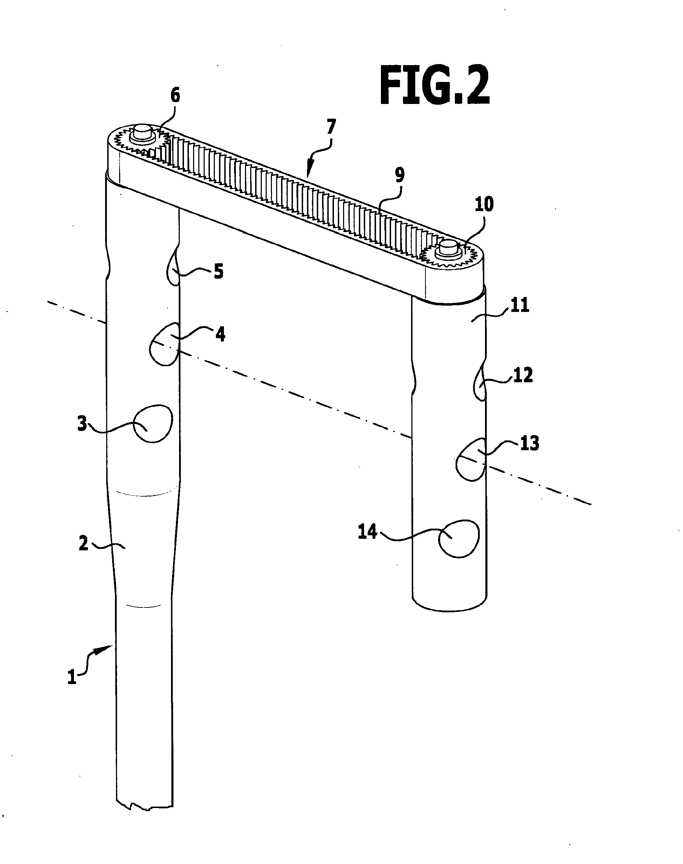

Der in den Figuren 1 und 2 dargestellte Markraumnagel 1 umfasst einen länglichen, im Wesentlichen kreiszylindrischen Schaft 2 mit mehreren diametral diesen durchsetzenden Durchgangsbohrungen 3, 4, 5, die üblicherweise in unterschiedlichen Winkelebenen bezüglich der Längsachse des Schaftes 2 verlaufen, also in unterschiedlichen Richtungen. Die Durchgangsbohrungen 3, 4, 5 können in einer Ebene verlaufen, die senkrecht zur Längsachse des Schaftes verläuft, sie können aber auch in einer zu dieser Ebene geneigten Ebene verlaufen. In dem dargestellten Ausführungsbeispiel sind jedoch nur solche Durchgangsbohrungen 3, 4, 5 dargestellt, die in Ebenen verlaufen, die senkrecht auf der Längsachse des Schaftes 2 stehen.

An seinem oberen Ende trägt der Schaft 2 in dem dargestellten Ausführungsbeispiel ein drehfest mit dem Schaft 2 verbundenes Zahnrad 6 sowie einen radial von dem Schaft 2 abstehenden Verbindungssteg 7, der mit dem Schaft 2 frei drehbar verbunden ist, die Drehachse des Verbindungssteges 7 fällt dabei mit der Mittelachse des Zahnrades 6 zusammen.

Der Verbindungssteg 7 bildet ein Gehäuse aus mit einem in der Zeichnung nicht dargestellten Unterteil und einem auf diesen aufsetzbaren Deckel 8. In dem Innenraum dieses Gehäuses wird das Zahnrad 6 des Schaftes 2 aufgenommen und außerdem ein Zahnriemen 9, der das Zahnrad 6 umschlingt und der am anderen Ende des Verbindungssteges 7 um ein weiteres Zahnrad 10 herumläuft. Dieses Zahnrad 10 ist drehfest verbunden mit einem stabförmigen Zielkörper 11, der parallel zum Oberteil des Schaftes 2 angeordnet ist und der relativ zu dem Verbindungssteg 7 um eine Drehachse frei verdrehbar ist, die parallel zu der Drehachse des Verbindungssteges 7 am Schaft 2 verläuft.

In dem Zielkörper 11 sind mehrere diesen diametral durchsetzende Führungskanäle 12, 13, 14 angeordnet, und zwar derart, dass deren Winkellage relativ zu der Drehachse des Zielkörpers 11 identisch ist mit der Winkellage der Durchgangsbohrungen 3, 4, 5 in dem Schaft 2.

Wenn man den Schaft 2 in einen Röhrenknochen einsetzt, ragt sein oberes Ende aus diesem hervor, so dass der Verbindungssteg 7 um die Längsachse des Schaftes 2 gegenüber diesem verdreht werden kann. Dies führt dazu, dass durch das Kämmen des Zahnriemens 9 mit den Zahnrädern 6 und 10 bei einer solchen Verdrehung des Verbindungssteges 7 synchron und zwangsläufig auch der stabförmige Zielkörper 11 um seine Drehachse verdreht wird, d.h. gegenüber dem Verbindungssteg 7 weisen der Schaft 2 und der Zielkörper 11 immer dieselbe Winkellage auf, d.h. unabhängig von der Stellung des Verbindungssteges 7 bleiben der Schaft 2 und der Zielkörper 11 immer in der gleichen Winkelstellung, und dies bedeutet natürlich auch, dass damit die Achsen der Durchgangsbohrungen 3, 4, 5 des Schaftes 2 einerseits und die Achsen der Führungskanäle 12, 13, 14 des Zielkörpers 11 andererseits immer parallel zueinander bleiben.

In einer bestimmten Stellung des Verbindungssteges 7, wenn dieser nämlich mit der Längsachse einer Durchgangsbohrung 3 ausgerichtet ist, ist damit zwangsläufig auch der entsprechende Führungskanal mit dieser Durchgangsbohrung ausgerichtet. Dies ist am Beispiel der Figur 1 für die oberste Durchgangsbohrung 3 und den obersten Führungskanal 2 gezeigt, im Ausführungsbeispiel der Figur 2 dagegen bei einer anderen Winkelstellung des Verbindungssteges 7 gegenüber dem Schaft 2 für die mittlere Durchgangsbohrung 4 und den mittleren Führungskanal 13. Der Benutzer kann allein durch Verdrehung des Verbindungssteges 7 gegenüber dem Schaft 2 auswählen, welche der Durchgangsbohrungen mit einem Führungskanal im Zielkörper ausgerichtet wird. Es ist auch leicht zu erkennen, wo eine Ausrichtung erfolgt, ausgerichtet ist nämlich immer der Führungskanal, der direkt auf den Schaft 2 zeigt, der also radial zum Schaft 2 verläuft. Dies erleichtert dem Benutzer das Einführen eines Werkzeuges, beispielsweise eines Bohrers. Besondere Kennzeichnungen der Führungskanäle sind daher in der Regel nicht notwendig, weil allein durch die Ausrichtung eines Führungskanals auf den Schaft 2 hin erkennbar ist, dass dieser Führungskanal mit einer entsprechenden Durchgangsbohrung ausgerichtet ist.

Auf diese Weise kann der Benutzer sehr einfach die entsprechenden Bohrungen in den Knochen einbringen und danach den Verbindungssteg 7 und den Zielkörper 11 von dem Schaft 2 abnehmen. Diese sind zu diesem Zweck lösbar mit dem Schaft verbunden, das kann im übrigen auch für das Zahnrad 6 gelten, das gegebenenfalls auch vom Schaft 2 abgenommen werden kann, so dass zur Fixierung von Knochenteilen nach einem Eingriff nur der Schaft 2 im Körper verbleibt.

## Patentansprüche

1. Markraumnagel (1) mit einem in den Markraum eines Röhrenknochens einsetzbaren Schaft (2), der mehrere diametrale Durchgangsbohrungen (3, 4, 5) mit unterschiedlicher Winkellage in Bezug auf die Längsachse des Schaftes (2) aufweist, und mit einem über eine Halterung mit dem Schaft (2) verbundenen, im Abstand zu diesem angeordneten Zielkörper (11), der Führungskanäle (12, 13, 14) für ein Werkzeug aufweist, die mit jeweils einer Durchgangsbohrung (3, 4, 5) des Schaftes (2) ausgerichtet sind, **dadurch gekennzeichnet, dass** die Halterung (7) des Schaftes (2) um die Längsachse des Schaftes (2) drehbar mit diesem und um eine parallele Drehachse drehbar mit dem Zielkörper (11) verbunden ist, dass Getriebemittel (9, 10, 6) vorgesehen sind, die bei einer Drehung der Halterung (7) relativ zum Schaft (2) gleichzeitig den Zielkörper (11) relativ zur Halterung (7) um denselben Drehwinkel verdrehen, so dass der Schaft (2) und der Zielkörper (11) bei allen Winkelstellungen der Halterung (7) immer zueinander eine unveränderte Winkelstellung beibehalten, und dass die Führungskanäle (12, 13, 14) in dem Zielkörper (11) winkelmäßig gleich orientiert sind wie die Durchgangsbohrungen (3, 4, 5) in dem Schaft (2).

2. Markraumnagel nach Anspruch 1, **dadurch gekennzeichnet, dass** die Getriebemittel einen Riemen (9) umfassen, der an der Halterung (7) angeordnet ist und um Riemenumlenkflächen (6, 10) herumläuft, die drehfest mit dem Schaft (2) bzw. dem Zielkörper (11) verbunden sind.

3. Markraumnagel nach Anspruch 2, **dadurch gekennzeichnet, dass** der Riemen (9) als Zahnriemen ausgebildet ist, dessen Zähne mit Zähnen auf den Riemenumlenkflächen (6, 10) kämmen.

4. Markraumnagel nach Anspruch 2, **dadurch gekennzeichnet, dass** die Riemen als Seilzüge ausgebildet sind.

5. Markraumnagel nach Anspruch 2, **dadurch gekennzeichnet, dass** die Riemen als Ketten ausgebildet sind.

6. Markraumnagel nach Anspruch 2, **dadurch gekennzeichnet, dass** die Riemen als Kugeldraht ausgebildet sind.

7. Markraumnagel nach Anspruch 1, **dadurch gekennzeichnet, dass** die Getriebemittel miteinander kämmende Zahnkränze umfassen.

8. Markraumnagel nach einem der voranstehenden Ansprüche, **dadurch gekennzeichnet, dass** die Halterung (7) ein Gehäuse ausbildet, in dem die Getriebemittel (6, 9, 10) angeordnet sind.

9. Markraumnagel nach einem der voranstehenden Ansprüche, **dadurch gekennzeichnet, dass** der Zielkörper (11) die Form eines Stabes hat, dessen Längsachse mit der Drehachse des Zielkörpers (11) relativ zu der Halterung (7) zusammenfällt.

## Claims

1. An intramedullary nail (1) having a shank (2), insertable in the medullary space of a long bone, which has a plurality of diametral through bores (3, 4, 5) at differing angular positions in relation to the longitudinal axis of the shank (2), and having a target body (11), connected to the shank (2) via a securing means and arranged at a distance from the shank (2), which has guide channels (12, 13, 14), aligned with a respective through bore (3, 4, 5) of the shank (2), for a tool, **characterised in that** the securing means (7) of the shank (2) are connected to the shank (2) so as to be rotatable about the longitudinal axis of the latter and are connected to the target body (11) so as to be rotatable about a rotational axis parallel to the longitudinal axis of the shank (2), **in that** gearing means (9, 10, 6) are provided, which, upon rotation of the securing means (7) relative to the shank (2), simultaneously rotate the target body (11) relative to the securing means (7) by the same angle of rotation, so that the shank (2) and the target body (11) always maintain an unchanged angular position in relation to one another in all angular positions of the securing means (7), and **in that** the guide channels (12, 13, 14) in the target body (11) are in terms of angle oriented identically to the through bores (3, 4, 5) in the shank (2).

2. An intramedullary nail according to claim 1, **characterised in that** the gearing means comprise a belt (9) which is arranged on the securing means (7) and which runs around belt deflection surfaces (6, 10) connected in a rotationally-fixed manner to the shank (2) and the target body (11) respectively.

3. An intramedullary nail according to claim 2, **characterised in that** the belt (9) is in the form of a toothed belt whose teeth mesh with teeth on the belt deflection surfaces (6, 10).

4. An intramedullary nail according to claim 2, **characterised in that** the belts are in the form of cable lines.

5. An intramedullary nail according to claim 2, **characterised in that** the belts are in the form of chains.

6. An intramedullary nail according to claim 2, **characterised in that** the belts are in the form of ball wire.

7. An intramedullary nail according to claim 1, **characterised in that** the gearing means comprise toothed rings meshing with one another.

8. An intramedullary nail according to any one of the preceding claims, **characterised in that** the securing means (7) forms a housing in which the gearing means (6, 9, 10) are arranged.

9. An intramedullary nail according to any one of the preceding claims, **characterised in that** the target body (11) has the form of a rod whose longitudinal axis coincides with the rotational axis of the target body (11) relative to the securing means (7).

## Revendications

1. Clou d'ostéosynthèse intramédullaire (1) comprenant un corps allongé (2) qui peut être inséré dans la cavité médullaire d'un os long et présente plusieurs alésages de passage (3, 4, 5) d'orientation diamétrale et de position angulaire différente par rapport à l'axe longitudinal du corps allongé (2), et comprenant également un corps formant gabarit de pointage (11) qui est relié, par l'intermédiaire d'un support (7), au corps allongé (2), est agencé à distance de ce dernier et présente des canaux de guidage (12, 13, 14) destinés à un outil et alignés respectivement avec un alésage de passage (3, 4, 5) du corps allongé (2), **caractérisé en ce que** le support (7) du corps allongé (2) est relié avec ce dernier de manière rotative autour de l'axe longitudinal du corps allongé (2), et est relié de manière rotative autour d'un axe de rotation parallèle, avec le corps formant gabarit de pointage (11), **en ce que** sont prévus des moyens de transmission (9, 10, 6), qui, lors d'une rotation du support (7) par rapport au corps allongé (2), font tourner simultanément, du même angle de rotation, le corps formant gabarit de pointage (11) par rapport au support (7), de sorte que le corps allongé (2) et le corps formant gabarit de pointage (11) conservent toujours l'un par rapport à l'autre une position angulaire invariable pour toutes les positions angulaires du support (7), et **en ce que** les canaux de guidage (12, 13, 14) dans le corps formant gabarit de pointage (11) sont, sur le plan angulaire, orientés de la même manière que les alésages de passage (3, 4, 5) dans le corps allongé (2).

2. Clou d'ostéosynthèse intramédullaire selon la revendication 1, **caractérisé en ce que** les moyens de transmission comprennent une courroie (9) qui est agencée sur le support (7) et s'étend autour de surfaces de renvoi de courroie (6, 10) reliées de manière fixe en rotation respectivement au corps allongé (2) et au corps formant gabarit de pointage (11).

3. Clou d'ostéosynthèse intramédullaire selon la revendication 2, **caractérisé en ce que** la courroie (9) est réalisée sous forme de courroie crantée, dont les crans engrènent avec des dents sur les surfaces de renvoi de courroie (6, 10).

4. Clou d'ostéosynthèse intramédullaire selon la revendication 2, **caractérisé en ce que** les courroies sont réalisées sous forme de câbles de transmission.

5. Clou d'ostéosynthèse intramédullaire selon la revendication 2, **caractérisé en ce que** les courroies sont réalisées sous forme de chaînes.

6. Clou d'ostéosynthèse intramédullaire selon la revendication 2, **caractérisé en ce que** les courroies sont réalisées sous forme de fil à billes.

7. Clou d'ostéosynthèse intramédullaire selon la revendication 1, **caractérisé en ce que** les moyens de transmission comprennent des couronnes dentées engrenant mutuellement.

8. Clou d'ostéosynthèse intramédullaire selon l'une des revendications précédentes, **caractérisé en ce que** le support (7) forme un carter ou boitier dans lequel sont agencés les moyens de transmission (6, 9, 10).

9. Clou d'ostéosynthèse intramédullaire selon l'une des revendications précédentes, **caractérisé en ce que** le corps formant gabarit de pointage (11) présente la forme d'une tige dont l'axe longitudinal coïncide avec l'axe de rotation du corps formant gabarit de pointage (11) par rapport au support (7).
